# EUROPEAN PATENT SPECIFICATION

(11) **EP 3 801 759 B1**
(45) Date of publication and mention of the grant of the patent: **03.01.2024**
(21) Application number: 19721639.3
(22) Date of filing: 09.05.2019
(51) Int. Cl.: A61N 5/10, G16H 50/50, A61B 18/08, A61B 18/18, A61N 1/40, A61B 34/00

(54) **A TEMPORAL THERMAL ABLATION REPRESENTATION FOR THERAPY DELIVERY**
ZEITLICHE THERMISCHE ABLATIONSDARSTELLUNG ZUR THERAPIEABGABE
REPRÉSENTATION D'ABLATION THERMIQUE TEMPORELLE POUR L'ADMINISTRATION D'UN TRAITEMENT

(30) Priority: 07.06.2018 EP 18176452
(43) Date of publication of application: 14.04.2021
(73) Proprietor: Koninklijke Philips N.V., 5656 AG Eindhoven (NL)
(72) Inventor: KUSTRA, Jacek Lukasz, 5656 AE Eindhoven (NL); HAUTVAST, Guillaume Leopold Theodorus Frederik, 5656 AE Eindhoven (NL); ELEVELT, Aaldert Jan, 5656 AE Eindhoven (NL); VAN DIJK, Edmond, 5656 AE Eindhoven (NL)
(74) Representative: Philips Intellectual Property & Standards
(86) International application number: PCT/EP2019/061884
(87) International publication number: WO 2019/233697

(56) References cited:
- WO-A2-2016/135584
- US-A1- 2008 033 419

## Description

### FIELD OF THE INVENTION

The present invention relates to a system for medical treatment, in particular in the field of thermal ablation treatment, a corresponding planning method and a respective computer program. More specifically, the invention relates to a system and a method for processing treatment data containing histograms as a function of therapy time.

### BACKGROUND OF THE INVENTION

Thermal therapy is a treatment modality increasingly used in cancer treatment in order to remove cancerous growths. In that respect, thermal ablation techniques are generally considered a good alternative to common surgery techniques as they are minimally invasive and thus allow to preserve as much of the surrounding biological tissue as possible.

Similar to related radiation therapy techniques, the goal in thermal ablation therapy is to efficiently deliver the thermal energy to the target area, such as tumors, while at the same time keeping the energy deposition in the organs at risk to a minimum. In order to achieve this goal, it is necessary to provide appropriate treatment planning and treatment monitoring i.e. to plan and monitor the time and delivery of the energy as accurately as possible.

In radiation therapy, such planning (and subsequent monitoring) is achieved by so-called dose-volume histograms (DVH). These DVHs are capable of visualizing the dose that may be absorbed in the target area and in the surrounding biological tissue, thus allowing to approximate the dose delivered to the target area, such as the tumor as well as the dose delivered to the organs at risk. Hereby, it is assumed that the energy deposited during irradiation is relatively constant over time. For radiation therapy, this is a valid assumption, i.e. it is sufficient to characterize the energy deposition in initial static terms. Thus, the DVH allow for relatively precise treatment planning and treatment monitoring in radiation therapy.

However, in contrast to radiation therapy, in thermal therapy, the dynamic behavior of the energy source during energy delivery has a stronger influence on the biological tissue response leading to ablation of the target area and, possibly, also in the organs at risk. That is, at a constant output energy of the source, the ablation of the tissue that is subject to the thermal ablation may vary e.g. depending on how quickly the energy is deposited and/or on the pattern of the energy deposition. In other words, in thermal ablation the main cause for cell destruction is not only the total deposited dose, but also the way how the dose is deposited. This dynamic behavior of the energy deposition has a strong influence on the efficiency and effects of the thermal treatment.

Accordingly, the employment of DVHs has proven to not provide a sufficient tool for treatment planning and/or treatment monitoring in thermal ablation treatment.

US 2008/0033419 A1 disclosesa thermal ablation system that is operable to perform thermal ablation using an x-ray system to measure temperature changes throughout a volume of interest in a patient. Image data sets captured by the x-ray system during a thermal ablation procedure provide temperature change information for the volume being subjected to the thermal ablation. Intermediate image data sets captured during the thermal ablation procedure may be fed into a system controller which may modify or update a thermal ablation plan to achieve volume coagulation necrosis targets. WO 2016/135584 A3 discloses a system for performing ablation that includes an ablation device configured to ablate tissue in accordance with control parameters and configured to make measurements during the ablation process. An imaging system is configured to measure an elastographic related parameter to monitor the ablation progress. A parameter estimation and monitoring module is configured to receive the measurements from the ablation device and/or the elastographic related parameter to provide feedback to adaptively adjust imaging parameters of the imaging device at different times during an ablation process.

### SUMMARY OF THE INVENTION

The invention is as defined in the appended claims. Embodiments, examples or aspects in the following disclosure, in particular treatment methods, which do not fall under the scope of the claims are presented for illustration purposes only and do not form part of the invention.

In order to provide a tool which allows thermal treatment planning and thermal treatment monitoring with high accuracy, it is necessary to account for the dynamic behavior of this kind of treatment. As such, the insufficiency of DVHs for planning and/or monitoring in thermal ablation treatment stems from the fact that these DVHs were initially designed to visualize radiation having a quasi-static behavior. Specifically, due to their cumulative nature, DVHs may only provide information about the cumulative energy deposited in the target area or the instantaneous temperature. The DVHs thus do not allow to track the evolution of the energy deposition during the treatment.

It is therefore an object of the invention to provide a system and a corresponding method which allows for an improved treatment planning and/or treatment monitoring process in thermal ablation treatment., More particularly, it is an object of the invention to provide a system and method which improves the accuracy of said planning and monitoring process. Even more specifically, it is an object of the invention to provide a system and method which allows to take into account the dynamic behavior of the energy source used in thermal ablation treatment.

This objective is achieved by a system for medical therapy comprising an input unit for receiving a time series of two-dimensional diagnostic diagrams, the diagnostic diagrams being indicative of the course of the thermal ablation therapy and representing at least one treatment parameter as a function of a dose delivered to a target area, each one of the time series of diagnostic diagrams obtained at a particular point in therapy time, a processing unit for processing the time series of diagnostic diagrams as a function of the therapy time to generate a time-resolved therapy summary, the generating comprising generating a three-dimensional dataset by merging the diagnostic diagrams as a function of the therapy time, and a display unit for generating and displaying a graphical representation of the time-resolved therapy summary, said graphical representation representing, in a first dimension, the at least one treatment parameter as a function of the therapy time in a second dimension and as a function of the dose in a third dimension.

In this context the term medical therapy may particularly refer to a therapy involving deposition of energy from an energy source exhibiting dynamic behavior in a target. Hereby, a system for medical therapy may particularly be any kind of system configured to allow planning and/or monitoring and/or controlling and/or assessment of the medical therapy performed. In some embodiments, the system may be configured as a planning system, an interventional system, a control system, a quality assurance system and/or a combination of any of these.

In some embodiments, the term medical therapy may particularly refer to thermal ablation therapy involving thermal ablation treatment. In this context, the term thermal ablation treatment may particularly refer to a treatment in which punctual heat is used to remove or destroy target tissue, such as tumors, in a target area. This may particularly be achieved by punctually supplying heat to a certain target tissue volume. In some embodiments, thermal needles and/or radiation are used to supply the heat to the target tissue volume. However, other commonly well-known methods may be used as well.

The term diagnostic diagram may particularly refer to a diagram indicative of the course of the medical treatment. More specifically, the term diagnostic diagram particularly refers to a two-dimensional indication of the energy deposition in the target area. Even more specifically, the plurality of diagnostic diagrams may particularly display at least one treatment parameter as a function of the dose deposited in the target area. In some embodiments, these treatment parameters may particularly comprise:
- Temperature
- Time at equivalent temperature
- Probability of ablation
- The ablation zone
- Tissue volume
- Input Power
- Thermal energy

Hereby, the temperature may particularly be an absolute temperature that may be measured in units of Kelvin, Celsius or Fahrenheit. In some embodiments, the temperature may be determined relative to the body temperature, the temperature at the start of therapy or as an absolute value. The temperature may relate to a temperature at the target area. Moreover, the temperature may also relate to a temperature at a heating element (like a needle) that is brought into close contact with the biological tissue.

The time at equivalent temperature may particularly be used as a parameter to indicate the time with its corresponding temperature in the target area, e.g. for therapy planning. The probability of ablation may particularly indicate the total probability that biological tissue in the target area will be ablated. In this context, the term ablation may be understood as a cell destruction or a removal of biological tissue from the main tissue body, specifically in the target area.

Further, the term ablation zone may specifically refer to a volume of tissue ablated or to the ablation volume with respect to a reference volume. Further, the term ablation zone may also be used to refer to the tissue under treatment, i.e. the tissue in the target area. The reference volume may be a cell volume or any predefined volume. It may be understood that the cell volume varies with the organs and tissue to be treated.

The term input power may refer to the power in units of energy (e.g. Watts, Joule, Calories) delivered by a power supply applied to the heating element, which may for example be a needle to deliver the heat to the tissue or a radiation source.

The diagnostic diagrams may comprise one or more values of one or more of the above-described parameters. In some embodiments, these parameters may be organized and/or visualized by means of one or more diagnostic diagrams. The organization and/or visualization by means of such diagnostic diagrams may particularly comprises to correlate the values of the above-cited parameters as a function of the dose delivered to the target area.

In this context, the term dose is to be interpreted broadly, i.e. to refer to the physical entity to be encoded for therapy planning and/or therapy monitoring, such as the energy delivered to the target area, the dose delivered to it or the like.

In some embodiments, the diagnostic diagrams may particularly comprise dose-volume histograms (DVHs). Usually, a DVH is a two-dimensional histogram including a one-dimensional curve representing the volume receiving a given dose as a function of said dose. In this context, it shall be understood that the term dose to be used in the context of the DVH may, again, be interpreted broadly and may particularly refer to the values as indicated herein above.

A time series of diagnostic diagrams may particularly refer to a plurality of diagnostic diagrams that have been obtained at different points in therapy time. Hereby, the term therapy time may particularly refer to the time of the treatment process. A point in therapy time may thus refer to a point in time during treatment. It shall be understood that during treatment may refer to the time of the actual treatment of a patient, i.e. to the time during which the medical treatment is performed. In this case, the time series of diagnostic diagrams may be used to provide a time-resolved therapy summary for treatment monitoring. The term during treatment may alternatively or additionally refer to the simulated time of the treatment, i.e. to the case where the treatment is planned. In that case the term therapy time refers to the fictional time point in case the therapy is performed as planned. The therapy time may be measured relative to the start of the (planned) treatment or may be the actual time during treatment. In some embodiments, the term treatment time may also refer to a fixed number of samples associated with inputs of the system or a flexible non-linear mapping associated with starting and stopping of the therapy device.

Each one of the diagnostic diagrams in the time series is representative of the energy deposition, by means of a treatment parameter as indicated herein above such as temperature, probability of ablation, thermal energy or the like, (and, thus, the treatment state) at the particular point in therapy time for which the diagnostic diagram obtained. In that context, it may be understood that the term obtaining may refer to the acquisition of a diagnostic diagram during treatment for treatment monitoring. Alternatively or additionally, the term may also refer to the computation of a diagnostic diagram at a supposed point in therapy time during the therapy for therapy planning. It shall be understood that the time interval between the points in therapy time at which the diagnostic diagrams may be obtained, and, thus, the time-resolution, are not limited to any particular value, but may vary depending on the application and purpose of the time-resolved therapy summary. To that end, the time interval may particularly be limited by certain factors such as the ability of the heating element for generating the thermal does to change temperature and to provide the corresponding thermal energy to the surrounding tissue and/or the biological processes underlying the thermal ablation and/or the thermal ablation time or the like. Accordingly, typical time intervals may lie in the order of magnitude between tenths of a second up to minutes. Hereby, the time intervals for therapy planning may be longer than the time intervals for therapy monitoring. That is, intervals in the range of minutes may particularly be used for therapy planning, whereas intervals in the range of use(sub-)seconds may be used for therapy monitoring. Other constellations may also be envisioned.

The time series of diagnostic diagrams may be processed by the processing unit to generate a time-resolved therapy summary.

In this context the term time-resolved therapy summary may particularly refer to a dataset in which the diagnostic diagrams have been merged as a function of the therapy time. That is, the time resolved summary comprises the information of the diagnostic diagrams as described above and also the information of the treatment time in chronological order. In this manner, the plurality of diagnostic diagrams may be summarized and arranged in a time-resolved manner that allows to indicate a correspondence of the treatment parameter indicated in the diagnostic diagram as a function of dose with the respective treatment time,. This allows to capture the dynamic nature of the medical therapy, and, in particular, follow the evolution of the treatment results over time. This allows for an improved therapy planning and monitoring in medical therapies in which the dynamic behavior of the therapy source may otherwise not be appropriately assessed.

In some embodiments, the system may further comprise an analyzation unit for analyzing the time-resolved therapy summary.

The analyzation unit may be configured to analyze and evaluate the time-resolved therapy summary comprising the diagnostic diagrams as a function of the therapy time. In some embodiments, the analyzation allows a treatment planning and/or treatment control system to automatically position and set up the therapy source to deliver the dose to the target area while minimizing the dose delivered to the organs at risk. In some embodiments, the time-resolved therapy summary is particularly used in thermal (ablation) therapy and allows to establish how to position the energy source in relation to the target area and to assess the time for which the source shall be used to deposit the thermal energy to said target area. Accordingly, the analyzation unit may be used to provide a therapy planning and/or therapy control system which may automatically perform appropriate treatment planning and/or control.

In some embodiments, the analyzation unit may optionally be configured to communicate with internal and/or external devices such as other therapy devices, data storage devices, specifically databases, further processing devices and/or display devices, to distribute the time-resolved therapy summary amongst them

In some embodiments, the system may further comprise a display unit for generating and displaying a graphical representation of the time-resolved therapy summary.

In this context, the term graphical representation may particularly relate to a two-dimensional graphical representation of the time-resolved therapy summary which allows to display at least one treatment parameter as represented in a single diagnostic diagram as a function of time. A graphical representation in accordance with the present invention may particularly be provided as a graphical representation of an ablatogram, i.e. as a representation of a time series of diagnostic diagrams as a function of time. Hereby, the second dimension of the diagnostic diagram, such as the dose, may be represented by means of a color coding.

In some embodiments, the graphical representation may particularly be a static diagram. That is, the treatment parameter represented by the time series of diagnostic diagrams may be represented in the graphical representation as a function of time, with the dose values represented by means of color coding, thereby giving an overview over the evolution of the treatment for a certain time frame (as constituted by the time axis).

In some embodiments, only real-time data is displayed in a dynamic graphical representation and is constantly updated during the course of the treatment. In this manner the therapy summary may be provided to a medical person in order to effectively perform medical treatment.

In some embodiments, the graphical representation may also be used to co-represent the real time dose and accumulated dose to achieve a better comparison between these values. In order to achieve this, several measures may be used as the accumulated dose representation, such as:
- Static/Average Tissue Damage Computation: Hereby, the delivered dose pattern is used to simulate tissue damage and present this information to the user
- Simulated tissue damage: The simulation of tissue damage may particularly be performed using image data inferring tissue properties. That is, the tissue properties that may be extracted from the image data are used to compute the simulated tissue damage and present the cumulative result to the user
- Total (thermal) energy delivered: Hereby, the accumulated (thermal) dose is directly represented in a similar manner than in radiation therapy.

The display unit may be part of the analyzation unit or communicatively connected to the analyzation unit. In some embodiments, the display unit may particularly be any kind of display screen, such as a TFT or LCD screen, configured to present the graphical representation to a user, such that the user may visually assess the results of the treatment planning and/or treatment monitoring process.

In some embodiments, each of the time-series of diagnostic diagrams represents at least one treatment parameter as a function of dose delivered to a target area. In some embodiments, the dose may comprise one or more of a thermal dose or an energy delivered to the target area. The at least one treatment parameter comprises one or more of a probability of ablation, an ablation distribution in a cell, an absorbing cell volume and/or an absorbed power by the cell.

In some embodiments, the diagnostic diagrams may particularly represent at least one treatment parameter as a function of the dose delivered to the area to be treated. Hereby, the term dose is to be interpreted broadly as indicated herein above. The term dose may particularly refer to the energy delivered to the target area or the dose delivered.

The treatment parameters may particularly comprise one or more of a temperature, a time at equivalent temperature, a probability of ablation, an ablation zone, a volume, a count value or a power value. In case of the ablation zone, only binary results may be achieved.

In some embodiments, a plurality of dose values of the dose delivered to the target area are represented in the graphical representation as color-coded values.

In accordance with some embodiments, the graphical representation of the time-resolved therapy summery may particularly inform the user about the dose delivered to the target area. This may be achieved by representing, in the graphical representation, at least one treatment parameter as a function of therapy time and by conveying a third dimension, namely the dose delivered at a particular point in therapy time by means of a color code. As an example, the color-coding may be performed by presenting the dose values using a color gradient running from blue to red where blue indicates a low dose value and red indicates a high dose value. The values indicated by the different colors may further be indicated by means of a separate color scale. This kind of representation may particularly be referred to as an ablatogram.

In some further embodiments, the system comprises a user interface allowing a user to interact with the graphical representation.

In some embodiments, the graphical representation may be adjusted in order to obtain more information. As an example, the color scale may be changed, such that a smaller dose range is covered but the resolution is increased, or that a higher dose range is covered reduced resolution. This adjustment may be performed by a user via a user interface. In some embodiments, the user scrolling over the color scale indicating the different values represented by the color may change the range and resolution of the color scale. In some embodiments, a sliding button may be used to change the range and resolution of the color scale. Other implementations may likewise be envisioned.

In some embodiments, the graphical representation may further be configured to represent a single diagnostic diagram as obtained during a particular point in therapy time in response to a respective user input. That is, the user may select a particular point in therapy time and the diagnostic diagram obtained at that point may be represented to the user as part of the graphical representation, for example by means of a pop-up window. In that respect, user selection of the point in therapy time may particularly be performed by the user indicating the point in therapy time in the graphical representation of the time-resolved therapy summary. Alternatively or additionally, the user may input, for example via a keyboard, a particular point in therapy time in a respective window and the input may prompt the graphical representation of the particular diagnostic diagram at that time point to appear. Other implementations of this feature may also be envisioned.

In this manner, it is possible to represent the treatment parameters, indicative of the medical treatment, as a function of dose in a time-resolved manner, i.e. to convey a three-dimensional representation of the time-resolved therapy summary on a two-dimensional screen thereby allowing a user to more efficiently capture the performed and/or planned treatment.

In some further embodiments, generating the graphical representation further comprises generating a first time-resolved summary of a first time series of diagnostic diagrams representing real-time data, generating a second time-resolved summary of a second time series of diagnostic diagrams representing planned, data and representing the first time-resolved summary and the second time-resolved summary alongside one another in the graphical representation. In some embodiments, the graphical representation may represent a combination of a first time-resolved therapy summary of a first time series of diagnostic diagrams representing a particular treatment parameter as currently obtained in real-time for a patient and of a second time-resolved therapy summary of a second time series of diagnostic diagrams representing the same treatment parameter as planned for the treatment session. That is, the graphical representation comprises both, real-time information and information about the planned treatment.

Hereby, the first time-resolved therapy summary and the second time-resolved therapy summary may particularly be represented in the graphical representation alongside one another. In some embodiments, this encompasses a graphical representation in which the first therapy summary representing the real-time data is provided in a first graphic and the second therapy summary representing the planning data is provided in a second graphic, whereby the first and second graphic are displayed alongside one another in the graphical representation, such as being displayed above one another or next to one another on the display screen. Hereby, the first graphic may particularly show a time-resolved therapy summary in which the diagnostic diagrams as obtained in real time during the therapy session are displayed as a function of the therapy time and in which the dose is encoded in terms of a color code. In a corresponding manner, the second graphic may show a time-resolved therapy summary in which the diagnostic diagrams according to the planned therapy are displayed as a function of the therapy time and in which the dose is encoded terms of a color code.

In some embodiments, the first and second time-resolved therapy summary may also be provided in a single graphic. More specifically the single graphic may also be used to represent at least one treatment parameter as a function of therapy time while including the (actually delivered and/or planned) dose in terms of a color code. In some embodiments, the first time-resolved therapy summary may particularly be represented at that part of the graphic which represents the therapy time that has already expired during the therapy session. The second time resolved summary may be represented at that part of the graphic which represents the therapy time that has not yet occurred, but will become relevant during the course of the therapy session. In this case, the graphical representation may particularly be dynamic, e.g. by means of a sliding window, and frequently be updated as the therapy time passes. Hereby, the portion of the graphical representation representing the first time-resolved therapy summary of the real-time data may grow while the portion representing the second time-resolved therapy summary of the planned data diminishes. This allows to provide an improved comparison of the planned and actually delivered dose, respectively.

Hereby, the first time-resolved therapy summary may particularly be generated based on real-time measurements of at least one treatment parameter during treatment, such as the volume, probability of ablation or the like, which are represented in respective diagnostic diagrams as a function of the dose delivered to the target area and/or to the organs at risk.

The second time-resolved therapy summary may be generated based on a simulation of the treatment process. In some embodiments, the simulation may be based on diagnostic image data obtained by means of a medical imaging modality, such as X-ray imaging, ultrasound imaging, magnetic resonance imaging or the like.

Hereby, the therapy time may particularly be the relative treatment time, relative to the beginning of the treatment session, measured in seconds, minutes or on another suitable time scale. The therapy time may also correspond to time of day, an absolute timing value measured during therapy, a fixed number of samples and/or a flexible non-linear mapping associated with the beginning and ending of the therapy session. To that end, a flexible non-linear mapping may particularly be used when it is more important to represent the peak temperature (or a like metric) along with the time spent at that temperature than to represent the actual pattern to reach said peak temperature. In those cases, a linear time representation may be used for the peak temperature, while the non-peak patterns in-between are represented by means of a non-linear time "compression" (i.e. are not linearly represented in the image). This non-linear representation, i.e. the linear representation of the peak patterns along with the non-linear representation of the remaining patterns, allows to represent longer timelines in a single image.

According to a further aspect a method for medical therapy is provided, the method comprising the steps of, receiving, at an input unit, a time series of two-dimensional diagnostic diagrams, the diagnostic diagrams being indicative of the course of the thermal ablation therapy and representing at least one treatment parameter as a function of a dose delivered to a target area, each one of the series of diagnostic diagrams obtained at a particular point in therapy time; processing, at a processing unit, the time series of diagnostic diagrams as a function of the therapy time to generate a time-resolved therapy summary, the generating comprising generating a three-dimensional dataset by merging the diagnostic diagrams as a function of the therapy time, and generating and displaying, at a display unit, a graphical representation of the time-resolved therapy summary, said graphical representation representing, in a first dimension, the at least one treatment parameter as a function of the therapy time in a second dimension and as a function of the dose in a third dimension.

In some embodiments, the method may further comprise receiving, via a user interface, a user interaction with the graphical representation.

According to a further aspect, a computer program for controlling the above described system, which, when executed, is adapted to perform the above described method. In an even further aspect, a computer readable medium is provided, the computer-readable medium having stored thereon the above computer program.

It shall be understood that the system for medical therapy treatment may be implemented by means of processing device. Hereby, the input unit, the processing unit, analyzation unit, the display unit and/or the user interface may be implemented as modules in the processing unit. The functionality of these modules may in particular be implemented by means of a respective algorithm. This algorithm may in particular be implemented using a machine learning algorithm implemented on said processing unit including said modules.

It shall be understood that the system of claim 1, the method of claim 11, the computer program of claim 13, and the computer-readable medium of claim 14 have similar and/or identical preferred embodiments, in particular, as defined in the dependent claims. It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically illustrates a system for medical therapy according to an embodiment.
Fig. 2 schematically illustrates a system for medical therapy according to a further embodiment.

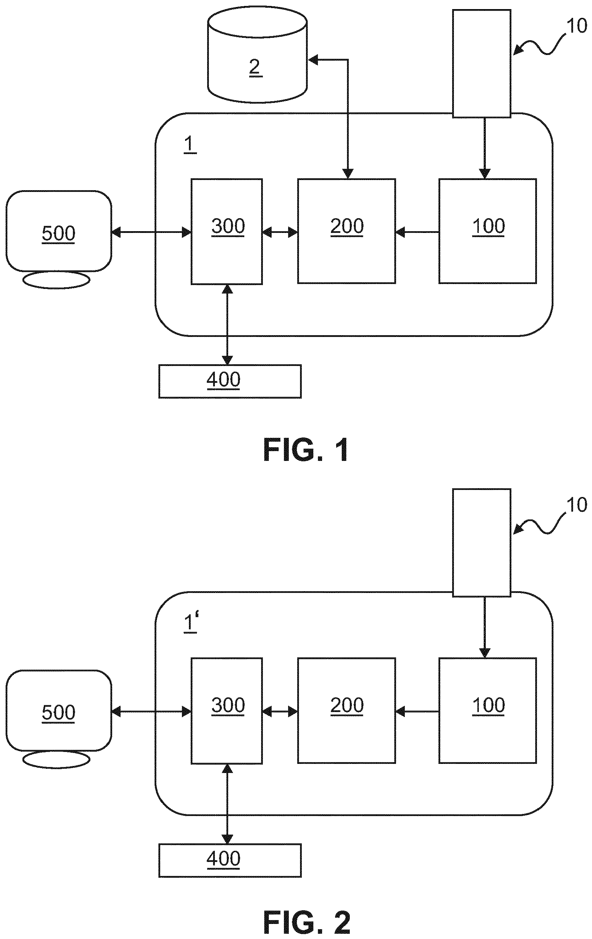

Fig. 2 schematically shows a method for generating a time-resolved therapy summary according to an embodiment.

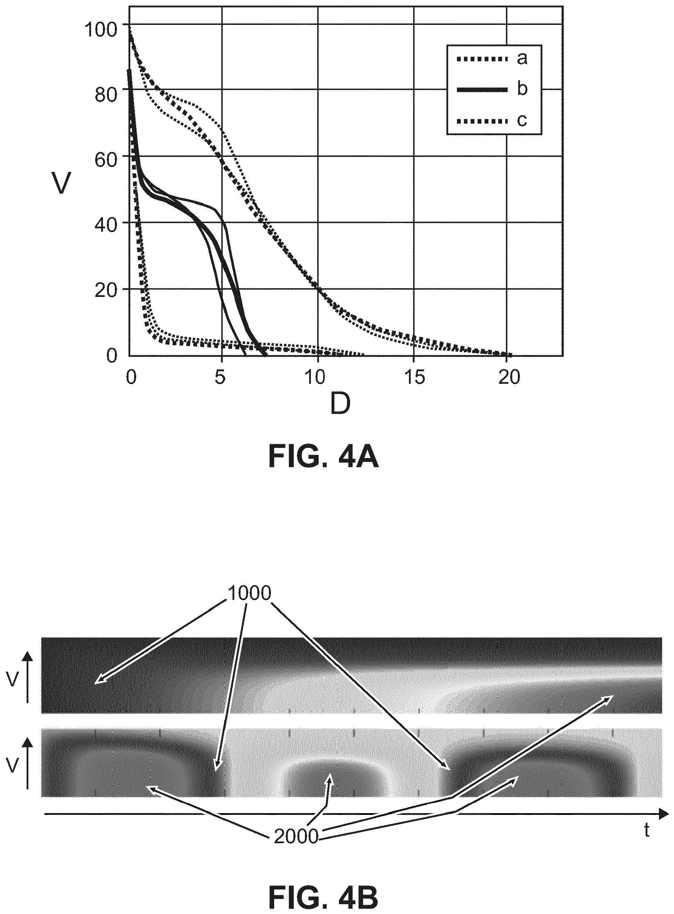

Figs. 4A schematically illustrates an exemplary diagnostic diagram according to an embodiment.
Fig. 4B schematically shows an exemplary graphical representation of a time-resolved therapy summary according to an embodiment.

### DETAILED DESCRIPTION OF EMBODIMENTS

The illustration in the drawings is schematically. In different drawings similar or identical elements are provided with the same reference numerals.

Fig. 1 illustrates a system 1 for medical treatment according to an exemplary embodiment. The system 1 comprises an input unit 100 which is configured to receive a time series of diagnostic diagrams 10, a processing unit 200 configured to process the time series of diagnostic diagrams 10, an analyzation unit 300, a user interface 400, and a display unit 500. Further, the system 1 is communicative coupled to external database 2.

In the exemplary embodiment of Fig. 1, the treatment to be performed corresponds to a thermal ablation treatment. The system 1 is hereby used for treatment monitoring. The input unit 100 may receive a first time series of diagnostic diagrams 10 which have been measured in real-time at a plurality of points in treatment time. In this particular example, each of the diagnostic diagrams represents the measured volume as a function of the thermal dose at that particular point in treatment time.

The input unit 100 may subsequently provide the first time series of diagnostic diagrams 10 to the processing unit 200. The processing unit 200 may then use the time series of diagnostic diagrams 10 to generate a first time resolved-therapy summary. That is, the processing unit 200 may merge each of the plurality of diagnostic diagrams from the first time series of diagnostic diagrams 10 as a function of time. Hereby, the first time-resolved summary also comprises the values of the thermal dose as represented by the individual diagnostic diagrams that have been merged in a time-resolved manner.

The system 1 may be communicatively coupled to the external database 2. In this particular embodiment of Fig. 1, the system 1 is coupled to external database 2 by means of the processing unit 200. From the external database 2 the processing unit may further receive a second time-resolved summary generated from a second time series of diagnostic diagrams, which have been simulated, based on diagnostic imaging data, for a plurality of points in treatment time. In this particular example, each of the second time series of diagnostic diagrams represents the simulated volume as a function of the thermal dose at that particular point in treatment time.

The processing unit 10 may then combine first time-resolved therapy summary and the second time-resolved therapy summary and forward the first and second time-resolved therapy summary to analyzation unit 300. Analyzation unit 300 may then analyze and evaluate the first and/or second time-resolved therapy summary. In the particular example according to Fig. 1, analyzation unit 300 compares the first time resolved therapy summary representing the treatment parameters collected in real-time with the second time resolved therapy summary representing the planned treatment parameters. In the exemplary embodiment of Fig. 1, analyzation unit 300 may be configured to output an indication in case the deviation between the first time-resolved therapy summary and the second time-resolved therapy summary becomes too severe. This indication may prompt a user to check whether the treatment is properly applied, thus preventing mistreatment of a patient.

The analyzation unit 300 may further be communicative coupled to a user interface 400 and a display unit 500. In the particular embodiment of Fig. 1, this user interface may particularly be a keyboard or the like. The display unit 500 may particularly be a display screen. In some embodiments, display unit 500 may be a touch screen, thereby providing an (additional) user interface to the user.

The display unit 500 may receive, from the analyzation unit 300, the first time-resolved therapy summary and the second time-resolved therapy summary and may generate a graphical representation thereof. In the embodiment according to Fig. 1, the display unit 500 particularly generates a graphical representation in terms of a sliding window, in which the first time-resolved therapy summary and the second time-resolved therapy summary are displayed alongside one another, whereby the first time-resolved therapy summary is graphically represented in the graphical presentation up to the present point in therapy time and the second time-resolved therapy summary is graphically represented starting with the next point following the present point in therapy time. The graphical representation is dynamically updated as the therapy time passes.

Based on the graphical representation on the display unit 500, a user, such as a medical personnel, may review the currently running treatment session and visually check whether everything is going according to plan. Further, using the user interface 400, the user may may interact with the graphical representation of the first and second time resolved therapy summary. This for example allows a user to select a particular point in therapy time which prompts the display unit 500 to generate a graphical representation of the diagnostic diagram 10 collected at and/or simulated for that particular point in therapy time and to present this representation to the user for further inspection.

The user may further indicate, via the user interface 400, that the first and/or second time resolved therapy summary shall be stored. In that case, the processing unit 200 is prompted to distribute the currently available first and second therapy summary to the external database 2. In some embodiments, the processing unit 200 may wait until the therapy session is finished and distribute the first and second time-resolved therapy summary only thereafter.

Fig. 2 schematically represents a system 1' for medical therapy according to another embodiment. In the exemplary embodiment of Fig. 2, system 1' is a therapy planning system. That is, the time series of diagnostic diagrams 10 that is received by system 1' comprises a plurality of diagnostic diagrams which have been simulated, for example in a manner as described herein above in relation to Fig. 1.

Upon receipt of the (simulated) time series of diagnostic image data 10 at the input unit 100, these are transferred to processing unit 200 and processed in the manner as described herein above in relation to Fig. 1 in order to generate a time-resolved therapy summary. The time-resolved therapy summary may then be forwarded to analyzation unit 300, which uses the time-resolved therapy summary to determine a potential treatment session for the patient for which the diagnostic diagrams have simulated, i.e. from which the diagnostic image data has been obtained. The analyzation unit 300 may then provide the information regarding the potential treatment, along with the time resolved therapy summary, to display unit 500.

The display unit 500 may then generate a graphical representation encompassing the time-resolved therapy summary of the planned therapy as well as the information for the proposed treatment session and output the graphical representation to a user. To that end, the information for the proposed treatment session may particularly comprise an indication of the target area, the positioning of the thermal energy source, the proposed treatment time or the like.

The user may then use the user interface 400 to review the proposed information and assess, in particular on the basis of the graphical representation of the time-resolved therapy summary, whether the analyzation units treatment plan is sufficient. The user may optionally edit the treatment plan where necessary. In some embodiments, the user may also directly accept the proposed treatment plan.

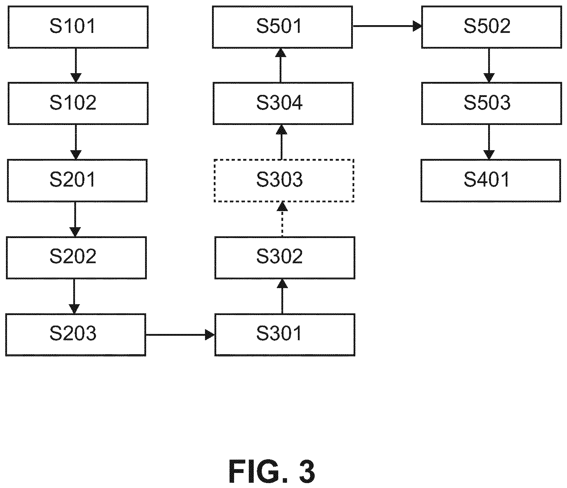

Fig. 3 schematically illustrates a method for generating and displaying a time-resolved therapy summary for a thermal ablation therapy treatment according to an embodiment.

In step S101, input unit 100 receives a time series of diagnostic diagrams 10. This time series of diagnostic diagrams may either be a time series which has been measured in real-time or may correspond to a simulated time series of diagnostic diagrams or may be both, depending on the purpose of the time-resolved therapy summary being used for either treatment monitoring or treatment planning or both. The time series of diagnostic diagrams comprises a plurality of diagnostic diagrams which have been obtained for a plurality of points in treatment time. In this particular example, each of the diagnostic diagrams represents the volume as a function of the thermal dose at that particular point in treatment time.

In step S 102, the input unit 100 provides the time series of diagnostic diagrams 10 to the processing unit 200. In step S201, the processing unit 200 receives the time series of diagnostic diagrams and uses them, in step S202, to generate a time-resolved therapy summary. In order to do so, the processing unit 200 merges each of the plurality of diagnostic diagrams 10 as a function of time, while maintaining the information about the thermal dose values as originally represented by each of the individual diagnostic diagrams 10.

In step S203, the processing unit 200 then forwards the thus generated time-resolved therapy summary to analyzation unit 300, where it is received at step S301.

In step S302, analyzation unit 300 evaluates the time-resolved therapy summary in order to determine a possible treatment plan and/or evaluate the ongoing treatment process. In case of any issues, the analyzation unit 300 may, in step S303, output an indication which may prompt a user to countercheck the proposed treatment plan and/or to intervene with the ongoing treatment process. It shall be understood that step S303 only occurs in case the analyzation unit 300 detects a problem. Else, the method proceeds to step S304, in which the analyzation unit 300 sends the time-resolved therapy summary to display unit 500, where it is received in step S501.

In step S502, the display unit 500 generates a graphical representation of the time-resolved therapy summary and presents said graphical representation to a user in step S503.

Based on the graphical representation on the display unit 500, a user can then visually check the time-resolved treatment summary and interact, via the user interface 400, with the time-resolved treatment summary, respectively the graphical representation thereof, in step S401. As an example, a user may provide a user input to adjust the time-resolved treatment summary or may select a particular point in therapy time shown therein, which leads to a graphical representation of the respective diagnostic diagram being displayed or the like.

Figs. 4A and 4B schematically illustrate an exemplary diagnostic diagram and an exemplary graphical representation of a time-resolved therapy summary according to an embodiment.

More specifically, Fig. 4A represents a diagnostic diagram that has been embodied as a Dose Volume Histogram (DVH) as commonly applied for radiation therapy treatment. In this kind of diagnostic diagram, the horizontal axis represents the dose, which in case of thermal ablation treatment, may particularly correspond to the thermal dose, but may also correspond to the energy deposited or the cell death probability, and the vertical axis represents the volume in the target area that has been irradiated by the thermal energy source. It may either be given as a percentage of the cell volume in units of [%] or as total adsorbing volume in units of a volume.

In the exemplary embodiment of Fig. 4A, curves a, b and c schematically represent the absorbing volume V as a function of the delivered thermal dose D. Hereby, the different curves may particularly represent different parts of the target area. Alternatively or additionally, different curves may also represent the tissue in the target area and the organs at risk, respectively, thereby allowing to analyze which dose/energy deposition in the target area results in which dose/energy deposition in the organs at risk. This allows for an improved treatment planning and treatment monitoring procedure.

Fig. 4B represents an exemplary graphical representation of the time-resolved therapy summary in the form of a two-dimensional, color-coded diagram.

In this particular exemplary embodiment according to Fig. 4B, the time-resolved therapy summary summarizes time-series of diagnostic diagrams according to Fig. 4A, i.e. representing the absorbing volume V as a function of the dose D, as a function of the therapy (or treatment) time t. Accordingly, the volume V in the target area is represented on the vertical axis of the two-dimensional diagram as a function of the therapy time t represented on the horizontal axis. The values for the thermal dose D are encoded by means of the color coding of the graphical representation. In this context, it shall be understood that the color coding may represent the accumulated dose determined by true integration, the accumulated dose determined with dedicate math, the instantaneous dose or the like. In the particular embodiment according to Fig. 4B, the instantaneous dose is represented by the color coding. Hereby, the color coding may be used to represent any kind of parameter indicative of the delivered dose as mentioned herein above, such as temperature, time at equivalent temperature, probability of ablation, volume, count, power, ablation zone or the like.

In the exemplary embodiment of Fig. 4B, two possible approaches for applying the thermal dose are represented.

In the upper graphical representation of the time-resolved therapy summary, only a small portion of the volume in the target area is heated. That is, the thermal treatment exhibits a hotspot. This may for example be achieved using a needle-shaped heat source to apply a thermal dose to specific location over time. At the beginning of treatment session, i.e. at the point where the therapy time is at zero, the temperate is substantially equal for the entire volume. Thus, no thermal dose is present in the entire volume and, thus, the color code represents a low thermal dose 1000. With ongoing therapy time, the location in the target area to which the heat source applies the thermal dose shows an increase in thermal dose, from a low thermal dose 1000 to a high thermal dose 2000. In terms of color coding this may be encoded by exhibiting a change from blue (low) to green to yellow to orange to red (high) over time for that particular hotspot in the volume.

In the lower graphical representation of the time-resolved therapy summary, the thermal dose is delivered in a cyclic manner, that is, it is cycled between warm and cold temperatures, such as used in cryotherapy around a particular location in the target area. As a result, the change between high dose 2000 and low dose 1000 (or high and low temperature in this case) occurs in a cyclic manner. This is reflected by several spots of high, respectively low values represented in the color coded ablatogram during the course of therapy time.

Although the above-described embodiments relate to methods for thermal ablation treatment it may be noted that those methods may also be applied to radiation therapy.

Further, it shall be understood that, although in the above-described embodiments, the system is implemented as part of a system for therapy planning and/or treatment monitoring, the system may likewise be implemented in other systems, such as a therapy control system (TCS) and/or a therapy verification system (TVS).

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like receiving of the series of diagnostic diagrams, processing of the time series of diagnostic diagrams as a function of the therapy time to generate a time-resolved therapy summary, generating and displaying a graphical representation, allowing a user to interact with the time-resolved summary and/or the diagnostic diagrams et cetera performed by one or several units or devices can be performed by any other number of units or devices. These procedures in accordance with the invention can hereby be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

A system for medical therapy comprising an input unit for receiving a time series of diagnostic diagrams, each one of the time series of diagnostic diagrams obtained at a particular point in therapy time, and a processing unit for processing the time series of diagnostic diagrams as a function of the therapy time to generate a time-resolved therapy summary is disclosed. By means of such a system the dynamics of a medical therapy may be efficiently mapped and implementation of medical therapy may be significantly simplified for a person skilled in the art.

## Claims

1. A system (1, 1') for thermal ablation therapy comprising
an input unit (100) for receiving a time series of two-dimensional diagnostic diagrams (10), the diagnostic diagrams (10) being indicative of the course of the thermal ablation therapy and representing at least one treatment parameter as a function of dose, each one of the time series of diagnostic diagrams (10) related to a particular point in therapy time;
a processing unit (200) for processing the time series of diagnostic diagrams (10) as a function of the therapy time to generate a time-resolved therapy summary, the generating comprising generating a three-dimensional dataset by merging the diagnostic diagrams (10) as a function of the therapy time, and
a display unit (500) for generating and displaying a graphical representation of the time-resolved therapy summary, said graphical representation representing, in a first dimension, the at least one treatment parameter as a function of the therapy time in a second dimension and as a function of the dose delivered at a particular point in therapy time in a third dimension by means of a colour code.

2. A system (1, 1') according to claim 1, further comprising an analyzation unit (300) for analyzing the time-resolved therapy summary.

3. A system (1, 1') according to claim 1, wherein the dose may comprise one or more of: a thermal dose or an energy delivered to the target area.

4. A system (1, 1') according to claim 1, wherein the at least one treatment parameter comprises one or more of: a probability of ablation, an ablation distribution in a cell, absorbing cell volume, an adsorbed power by the cell.

5. A system according to claim 1, wherein a plurality of dose values of the dose delivered to the target area are represented in the graphical representation as colour-coded values.

6. A system (1, 1') according to claim 1, further comprising a user interface (400) allowing a user to interact with the graphical representation.

7. A system according to claim 1, wherein generating the graphical representation comprises:
- generating a first time-resolved summary of a first time series of diagnostic diagrams (10) representing real-time data;
- generating a second time-resolved summary of a second time series of diagnostic diagrams (10) representing planned data; and
- representing the first time-resolved summary and the second time-resolved summary alongside one another in the graphical representation.

8. A system according to claim 1, wherein generating the graphical representation comprises analyzing the diagnostic diagrams (10) as a function of time.

9. A system according to claim 5, wherein the colour-coded values may be one or more of instantaneous values or accumulated values.

10. A system according to claim 1, wherein the therapy time may be one or more of: a time relative to the start of therapy, a time during therapy, a time of planned therapy, a fixed number of samples, a flexible non-linear mapping associated with starting and stopping therapy.

11. A thermal ablation therapy planning method comprising the steps of:
- receiving, at an input unit (100), a time series of two-dimensional diagnostic diagrams (10), the diagnostic (10) diagrams being indicative of the course of the thermal ablation therapy and representing at least one treatment parameter as a function of dose, each one of the time series of diagnostic diagrams (10) related to a particular point in therapy time;
- processing, at a processing unit (200), the time series of diagnostic diagrams (10) as a function of the therapy time to generate a time-resolved therapy summary, the generating comprising generating a three-dimensional dataset by merging the diagnostic diagrams as a function of the therapy time; and
generating and displaying, at a display unit (500), a graphical representation of the time-resolved therapy summary, said graphical representation representing, in a first dimension, the at least one treatment parameter as a function of the therapy time in a second dimension and as a function of the dose a function of the dose delivered at a particular point in therapy time in a third dimension by means of a colour code.

12. The method according to claim 11, further comprising:
receiving, via a user interface (400), a user interaction with the graphical representation.

13. A computer program for planning thermal ablation therapy which, when executed by a processing unit, is adapted to perform the method according to claim 11.

14. A computer-readable medium having stored thereon the computer program according to claim 13.

## Patentansprüche

1. System (1, 1') zur thermischen Ablationstherapie, umfassend:
eine Eingabeeinheit (100) zum Empfang einer Reihe von zweidimensionalen Diagnosediagrammen (10), wobei die Diagnosediagramme (10) den Verlauf der thermischen Ablationstherapie anzeigen und mindestens einen Behandlungsparameter in Abhängigkeit von der Dosis darstellen, wobei sich jedes der Zeitreihen der Diagnosediagramme (10) auf einen bestimmten Zeitpunkt der Therapiezeit bezieht;
eine Verarbeitungseinheit (200) zum Verarbeiten der Zeitreihe von Diagnosediagrammen (10) in Abhängigkeit von der Therapiezeit, um eine zeitaufgelöste Therapiezusammenfassung zu erstellen, wobei das Generieren das Erzeugen eines dreidimensionalen Datensatzes durch Zusammenführen der Diagnosediagramme (10) als Funktion der Therapiezeit umfasst, und
eine Anzeigeeinheit (500) zum Erzeugen und Anzeigen einer grafischen Darstellung der zeitaufgelösten Therapiezusammenfassung, wobei die grafische Darstellung in einer ersten Dimension den mindestens einen Behandlungsparameter als Funktion der Therapiezeit in einer zweiten Dimension, und als eine Funktion der zu einem bestimmten Zeitpunkt der Therapiezeit abgegebenen Dosis in einer dritten Dimension mittels eines Farbcodes darstellt.

2. System (1, 1') nach Anspruch 1, weiterhin umfassend eine Analyseeinheit (300) zur Analyse der zeitaufgelösten Therapiezusammenfassung.

3. System (1, 1') nach Anspruch 1, wobei die Dosis eines oder mehrere von Folgendem umfassen kann: eine thermische Dosis oder eine an den Zielbereich abgegebene Energie.

4. System (1, 1') nach Anspruch 1, wobei der mindestens eine Behandlungsparameter einen oder mehrere von Folgendem umfasst: eine Ablationswahrscheinlichkeit, eine Ablationsverteilung in einer Zelle, absorbierendes Zellvolumen, eine von der Zelle absorbierte Leistung.

5. System nach Anspruch 1, wobei mehrere Dosiswerte der an den Zielbereich abgegebenen Dosis in der grafischen Darstellung als farbcodierte Werte dargestellt werden.

6. System (1, 1') nach Anspruch 1, weiterhin umfassend eine Benutzerschnittstelle (400), die es einem Benutzer ermöglicht, mit der grafischen Darstellung zu interagieren.

7. System nach Anspruch 1, wobei das Erzeugen der grafischen Darstellung Folgendes umfasst:
- Erzeugen einer ersten zeitaufgelösten Zusammenfassung einer ersten Zeitreihe von Diagnosediagrammen (10), die Echtzeitdaten darstellen;
- Erzeugen einer zweiten zeitaufgelösten Zusammenfassung einer zweiten Zeitreihe von Diagnosediagrammen (10), die geplante Daten darstellen; und
- Darstellen der ersten zeitaufgelösten Zusammenfassung und der zweiten zeitaufgelösten Zusammenfassung nebeneinander in der grafischen Darstellung.

8. System nach Anspruch 1, wobei das Erzeugen der grafischen Darstellung das Analysieren der Diagnosediagramme (10) als Funktion der Zeit umfasst.

9. System nach Anspruch 5, wobei es sich bei den farbcodierten Werten um einen oder mehrere Momentanwerte oder Endwerte handeln kann.

10. System nach Anspruch 1, wobei die Therapiezeit eine oder mehrere der folgenden sein kann: eine Zeit relativ zum Beginn der Therapie, eine Zeit während der Therapie, eine Zeit der geplanten Therapie, eine feste Anzahl von Proben, eine flexible nichtlineare Zuordnung im Zusammenhang mit dem Starten und Stoppen der Therapie.

11. Planungsverfahren für eine thermische Ablationstherapie, umfassend die Schritte von:
- Empfangen einer Zeitreihe zweidimensionaler Diagnosediagramme (10) an einer Eingabeeinheit (100), wobei die Diagnosediagramme (10) den Verlauf der Thermoablationstherapie anzeigen und mindestens einen Behandlungsparameter als Funktion der Dosis darstellen, wobei sich jedes der Zeitreihen der Diagnosediagramme (10) auf einen bestimmten Punkt in der Therapiezeit bezieht;
- Verarbeiten der Zeitreihe diagnostischer Diagramme (10) als Funktion der Therapiezeit in einer Verarbeitungseinheit (200), um eine zeitaufgelöste Therapiezusammenfassung zu erstellen, wobei das Generieren das Erzeugen eines dreidimensionalen Datensatzes durch Zusammenführen der diagnostischen Diagramme als Funktion der Therapiezeit umfasst; und
- Erzeugen und Anzeigen einer grafischen Darstellung der zeitaufgelösten Therapiezusammenfassung auf einer Anzeigeeinheit (500), wobei die grafische Darstellung in einer ersten Dimension den mindestens einen Behandlungsparameter als Funktion der Therapiezeit in einer zweiten Dimension und als Funktion der Dosis eine Funktion der zu einem bestimmten Zeitpunkt der Therapiezeit abgegebenen Dosis in einer dritten Dimension mittels eines Farbcodes darstellt.

12. Verfahren nach Anspruch 11, weiterhin umfassend:
Empfangen einer Benutzerinteraktion mit der grafischen Darstellung über eine Benutzerschnittstelle (400) .

13. Computerprogramm zur Planung einer thermischen Ablationstherapie, das bei Ausführung durch eine Verarbeitungseinheit dazu geeignet ist, das Verfahren nach Anspruch 11 durchzuführen.

14. Computerlesbares Medium, auf dem das Computerprogramm nach Anspruch 13 gespeichert ist.

## Revendications

1. Système (1, 1') de thérapie d'ablation thermique comprenant
une unité d'entrée (100) pour recevoir une série de diagrammes de diagnostic bidimensionnels (10), les diagrammes de diagnostic (10) étant indicatifs du déroulement de la thérapie d'ablation thermique et représentant au moins un paramètre de traitement en fonction d'une dose, chaque diagramme de la série chronologique de diagrammes de diagnostic (10) étant lié à un moment donné de la durée de la thérapie;
une unité de traitement (200) pour traiter la série chronologique de diagrammes de diagnostic (10) en fonction de la durée de la thérapie pour générer un résumé de la thérapie résolu dans le temps, la génération consistant à générer un ensemble de données tridimensionnelles par fusion des diagrammes de diagnostic (10) en fonction de la durée de la thérapie, et
une unité d'affichage (500) pour générer et afficher une représentation graphique du résumé de la thérapie résolu dans le temps, ladite représentation graphique représentant, dans une première dimension, l'au moins un paramètre de traitement en fonction de la durée de la thérapie dans une deuxième dimension et en fonction de la dose administrée à un moment donné de la durée de la thérapie dans une troisième dimension au moyen d'un code couleur.

2. Système (1, 1') selon la revendication 1, comprenant en outre une unité d'analyse (300) pour analyser le résumé de la thérapie résolu dans le temps.

3. Système (1, 1') selon la revendication 1, dans lequel la dose peut comprendre un ou plusieurs parmi: une dose thermique ou une énergie administrée à la zone cible.

4. Système (1, 1') selon la revendication 1, dans lequel l'au moins un paramètre de traitement comprend un ou plusieurs parmi: une probabilité d'ablation, une distribution d'ablation dans une cellule, un volume cellulaire absorbant, une puissance adsorbée par la cellule.

5. Système selon la revendication 1, dans lequel une pluralité de valeurs de dose de la dose administrée à la zone cible sont représentées dans la représentation graphique sous forme de valeurs codées en couleurs.

6. Système (1, 1') selon la revendication 1, comprenant en outre une interface utilisateur (400) permettant à un utilisateur d'interagir avec la représentation graphique.

7. Système selon la revendication 1, dans lequel la génération de la représentation graphique consiste à:
- générer un premier résumé résolu dans le temps d'une première série chronologique de diagrammes de diagnostic (10) représentant des données en temps réel;
- générer un deuxième résumé résolu dans le temps d'une deuxième série chronologique de diagrammes de diagnostic (10) représentant des données planifiées; et
- représenter le premier résumé résolu dans le temps et le deuxième résumé résolu dans le temps l'un à côté de l'autre dans la représentation graphique.

8. Système selon la revendication 1, dans lequel la génération de la représentation graphique comprend l'analyse des diagrammes de diagnostic (10) en fonction du temps.

9. Système selon la revendication 5, dans lequel les valeurs codées en couleurs peuvent être une ou plusieurs valeurs instantanées ou valeurs accumulées.

10. Système selon la revendication 1, dans lequel la durée de la thérapie peut être l'un ou plusieurs parmi: une durée relative au début de la thérapie, une durée pendant la thérapie, une durée de thérapie planifiée, un nombre fixe d'échantillons, un mappage non linéaire flexible associé au début et à l'arrêt de la thérapie.

11. Procédé de planification d'une thérapie d'ablation thermique comprenant les étapes consistant à:
- recevoir, au niveau d'une unité d'entrée (100), une série chronologique de diagrammes de diagnostic bidimensionnels (10), les diagrammes de diagnostic (10) étant indicatifs du déroulement de la thérapie d'ablation thermique et représentant au moins un paramètre de traitement en fonction d'une dose, chaque diagramme de la série chronologique de diagrammes de diagnostic (10) étant lié à un moment donné de la durée de la thérapie;
- traiter, au niveau d'une unité de traitement (200), la série chronologique de diagrammes de diagnostic (10) en fonction de la durée de la thérapie pour générer un résumé de la thérapie résolu dans le temps, la génération consistant à générer un ensemble de données tridimensionnelles par fusion des diagrammes de diagnostic en fonction de la durée de la thérapie, et
- générer et afficher, au niveau d'une unité d'affichage (500), une représentation graphique du résumé de la thérapie résolu dans le temps, ladite représentation graphique représentant, dans une première dimension, l'au moins un paramètre de traitement en fonction de la durée de la thérapie dans une deuxième dimension et en fonction de la dose, en fonction de la dose administrée à un moment donné de la durée de la thérapie dans une troisième dimension au moyen d'un code couleur.

12. Procédé selon la revendication 11, consistant en outre à:
recevoir, via une interface utilisateur (400), une interaction utilisateur avec la représentation graphique.

13. Programme informatique pour planifier une thérapie d'ablation thermique qui, lorsqu'il est exécuté par une unité de traitement, est adapté pour mettre en œuvre le procédé selon la revendication 11.

14. Support lisible par ordinateur sur lequel est stocké le programme informatique selon la revendication 13.
